Europäisches Patentamt

(19) European Patent Office

Office européen des brevets

(11) **EP 0 762 108 A2**

(12) **EUROPEAN PATENT APPLICATION**

(43) Date of publication:
12.03.1997 Bulletin 1997/11

(51) Int. Cl.⁶: **G01N 21/35**, A61B 5/00

(21) Application number: 96113759.3

(22) Date of filing: 28.08.1996

(84) Designated Contracting States:
DE FR GB IT

(30) Priority: 30.08.1995 JP 246852/95

(71) Applicant: **Kyoto Dai-ichi Kagaku Co., Ltd.**
**Minami-ku, Kyoto 601 (JP)**

(72) Inventors:
• **Yamasaki, Yutaka,**
**Kyoto Dai-ichi Kagaku Co., Ltd.**
**Minami-ku, Kyoto 601 (JP)**

• **Sakai, Toshikatsu,**
**Kyoto Dai-ichi Kagaku Co., Ltd.**
**Minami-ku, Kyoto 601 (JP)**
• **Sakura, Takeshi,**
**Kyoto Dai-ichi Kagaku Co., Ltd.**
**Minami-ku, Kyoto 601 (JP)**
• **Ashibe, Emi,**
**Kyoto Dai-ichi Kagaku Co., Ltd.**
**Minami-ku, Kyoto 601 (JP)**

(74) Representative: **Schoppe, Fritz, Dipl.-Ing.**
**Patentanwalt,**
**Georg-Kalb-Strasse 9**
**82049 Pullach (DE)**

(54) **Method of and apparatus for measuring ketone concentration in organism**

(57) A light source part (2) emits light of a plurality of wavelengths of a near infrared region, and a spectroscopic part (8) selects a wavelength which is subjected to absorption by a ketone body to be measured as a measuring wavelength from the measuring light of a plurality of wavelengths emitted from the light source part (2). A probe (4) comes into contact with an organism measuring portion (1) and irradiates the organism measuring portion (1) with the measuring light of the wavelength selected in the spectroscopic part (8), and a photoreceiving part (6) detects transmitted/scattered light of the measuring light incident upon the organism measuring portion (1). A light signal detected by the photoreceiving part (6) is converted to absorbance by a signal processing part (14), so that the concentration of the ketone body is calculated by a host computer (16) serving as an arithmetic • control part and displayed on a display part (18). No reagent is required, while the intravascular ketone body concentration is noninvasively measured.

Fig. 8

## Description

BACKGROUND OF THE INVENTION

Field of the Invention

The present invention relates to a method of and an apparatus for measuring a ketone body in organism, particularly at least one ketone body among acetoacetic acid, β-hydroxybutyric acid and acetone.

Description of the Background Art

A ketone body concentration in organism serves as a monitor indicating an effect of alimentotherapy for weight reduction and is used as an index of ketoacidosis of severe diabetes mellitus as well as an index for distinguishing insulin dependent diabetes mellitus from insulin independent diabetes mellitus.

It is known that, when lipid is intravitally used as an energy source, a ketone body is intravascularly accumulated as its metabolite. It is now being clarified that the effect of obesity treatment can be monitored by employing the intravascular ketone body as an index.

In a severe diabetic, lipid may be preferentially used as en energy source in place of carbohydrate to result in ketoacidosis of acidifying the blood due to increase of the intravascular ketone body concentration, such that the diabetic goes into a coma.

It is recognized that insulin dependent diabetes mellitus has such a tendency that the intravascular ketone body concentration is high as compared with the insulin independent diabetes mellitus.

In general, the intravascular ketone body concentration is detected through enzymologic color reaction from target blood.

A method of measuring a ketone body in urine or expiration is known as a method of noninvasively measuring an intravascular ketone body.

The measuring method directed to the blood requires blood collection followed by pain or a possibility of infection during the blood collection, while the measuring operation is complicated with requirement for a reagent.

While a test paper method is employed in the method of measuring a ketone body in urine, which is a noninvasive measuring method, a reagent is necessary. The method of measuring a ketone body in expiration utilizes gas chromatography or a gas detecting tube method, with requirement for large-scale apparatus and consumed products.

It is recognized that a ketone body concentration in urine or expiration has a time lag with respect to an intravascular ketone body concentration.

SUMMARY OF THE INVENTION

An object of the present invention is to provide a simple method of measuring an intravascular ketone body concentration which can noninvasively measure the intravascular ketone body concentration with neither requirement for a reagent nor time lag, and a measuring apparatus employed for the method.

According to the present invention, an organism is irradiated with near infrared light, so that the intravital ketone body concentration is quantitatively analyzed through light transmitted/scattered through/from the organism.

The term "transmitted/scattered" means such a phenomenon that light enters a light scattering target substance and goes out from the target substance, and hence a term "transmitted/scattered light" includes both of the so-called transmitted light going out in the direction of incidence of the light and the so-called reflected light going out in a direction opposite to the direction of incidence.

According to an aspect of the present invention, a light intensity at a measuring wavelength subjected to absorption by a ketone body in a near infrared region is detected from the light transmitted/scattered through/from the organism, so that the intravital ketone body concentration is quantitatively analyzed on the basis of the light intensity.

In order to guide a calibration expression for obtaining a ketone body concentration from an obtained absorption spectrum, it is necessary to remove interference by another component of an organism measuring portion. As one of such methods, there is a method employing multivariate analysis. In a multivariate analytical operation, multivariate regression analysis such as principal component regression analysis (PCR) or partial least square fitting (PLS) is employed for performing data analysis. Regression analysis can be made by employing a number of spectral intensities at once in the multivariate regression analysis, whereby quantitative analysis in higher accuracy is possible as compared with single regression analysis. While multiple regression analysis is most generally employed, a number of samples are necessary and its quantitative analytical accuracy is reduced if correlation between spectral intensities at respective wavenumbers is high. On the other hand, PCR which is multivariate regression analysis can intensify spectral intensities in a plurality of wavenumber regions to principal components which are irrelevant to each other while deleting unnecessary noise data, whereby high quantitative analytical accuracy can be attained. Further, PLS can also utilize sample concentration data in case of extracting a principal component, whereby high quantitative analytical accuracy can be attained similarly to PCR. As to the multivariate regression analysis, "Tahenryo Kaiseki" (by Kazuo Nakatani, Shinyo-Sha, Japan) can be referred to.

In order to extract necessary information from a spectrum complicatedly fluctuating by various fluctuation factors, data processing by a computer is remarkably useful. A typical processing method is stored in processing software which is provided on a commercially available near infrared apparatus or the like. An example of such commercially available software is "Unscramber" by CAMO Company. Typical processing methods are the aforementioned multiple regression analysis, PLS, the principal component regression analysis and the like.

Large flows of data processing which is applied to quantitative analysis are (1) formation of a calibration model, (2) evaluation of the calibration model, and (3) determination of an unknown sample.

In order to perform calibration, it is necessary to measure a proper number of samples for forming a calibration curve in sufficient accuracy. Obtained spectra are pretreated at need. Typical pretreatments are smoothing of spectra, differential and normalization, which are general processing methods.

The calibration is processing of constructing a numerical relational expression between spectral data and an analytical value of a target characteristic, i.e., a model. The model is formed by a statistical technique with analytical values of the samples for forming a calibration curve and the spectral data.

In order to correctly evaluate accuracy of prediction of the formed calibration curve with respect to an unknown sample, a measurement error with respect to the unknown sample is obtained through an evaluation sample. If a determination is made that the accuracy of the calibration curve is insufficient, the type of the processing method or parameters are changed at need, for correcting the calibration curve.

A calibration curve which is regarded as having sufficient accuracy is employed as a relational expression for predicting the value of the target characteristic from the spectral data in analysis of the unknown sample, for determining the unknown sample concentration.

In case of applying multivariate analysis to the inventive method, light intensities at measuring wavelengths are detected respectively from light transmitted/scattered through/from an organism on the assumption that a plurality of wavelengths subjected to absorption by a ketone body in a near infrared region are the measuring wavelengths, so that the intravital ketone body concentration is quantitatively analyzed from the light intensities through multivariate analysis.

The measuring wavelengths are wavelengths whose correlation coefficients R between ketone body concentrations and absorbance values are at least 0.8, preferably at least 0.9, in aqueous ketone body solution measurement. Each correlation coefficient R is calculated as follows:

$$R = \frac{\sum\limits_{i=1}^{n} \{(x_i - X)(y_i - Y)\}}{\sqrt{[\sum\limits_{i=1}^{n} (x_i - X)^2][\sum\limits_{i=1}^{n} (y_i - Y)^2]}}$$

where $x_i$ represents the concentration of each component at each point, $y_i$ represents absorbance with respect to $x_i$, X represents the mean value of the concentration of each component, and Y represents the mean value of the absorbance.

The ketone body to be measured includes at least one component among acetoacetic acid, β-hydroxybutyric acid and acetone, and a measuring wavelength for each component belongs to a near infrared region expressed in wavenumbers of 10000 to 4000 cm$^{-1}$. In more concrete terms, the wavelength is selected from around 6400 to 5400 or 4800 to 4300 cm$^{-1}$ for acetoacetic acid, from around 7600 to 5600 or 4800 to 4200 cm$^{-1}$ for β-hydroxybutyric acid, or from around 7800 to 5500 or 4800 to 4200 cm$^{-1}$ for acetone.

A measuring apparatus according to another aspect of the present invention comprises a light source part for emitting light of a plurality of wavelengths in a near infrared region, a probe which comes into contact with an organism measuring portion for irradiating the organism measuring portion with the light from the light source part as measuring light, a photoreceiving part for detecting transmitted/scattered light of the measuring light received from the organism measuring portion, a spectroscopic part which is provided on an optical path for irradiating the organism measuring portion with the measuring light or that for introducing the transmitted/scattered light of the measuring light into the photoreceiving part for selecting one or a plurality of wavelengths subjected to absorption by a ketone body to be measured and selected as a measuring wavelength or measuring wavelengths from the measuring light of the plurality of wavelengths emitted from the light source part, and an arithmetic part for calculating the ketone body concentration on the basis of a light intensity or light intensities at the measuring wavelength(s) detected by the photoreceiving part.

In a measuring apparatus according to still another aspect of the present invention, it is also possible to use a plurality of types of laser units, laser diodes or light emitting diodes, having different emission wavelengths, for emitting wavelength light of a near infrared region subjected to absorption by a ketone body to be measured as a light source part. When a plurality of laser diodes or light emitting diodes are employed as the light source part, no spectroscopic part may be employed but the laser diodes or the light emitting diodes may be successively switched and turned on.

According to the inventive method, no reagent is required, and the intravascular ketone body concentration can be noninvasively measured. Consequently, it is possible to readily recognize the degree of weight reduction, and diabetes mellitus can be quickly diagnosed.

The foregoing and other objects, features, aspects and advantages of the present invention will become more apparent from the following detailed description of the resent invention when taken in conjunction with the accompanying drawings.

## BRIEF DESCRIPTION OF THE DRAWINGS

Fig. 1 illustrates absorption spectra of aqueous β-hydroxybutyric acid solutions in a near infrared region;

Fig. 2 illustrates a calibration curve obtained by plotting absorbance values of aqueous β-hydroxybutyric acid solutions at an absorption wavenumber of 4355.8 $cm^{-1}$ with respect to concentrations;

Fig. 3 illustrates results obtained by calculating correlation coefficients R of the absorbance values and the concentrations from the absorption spectra of Fig. 1 as to respective wavenumbers;

Fig. 4 illustrates results obtained by primarily differentiating parts of the absorption spectra shown in Fig. 1;

Fig. 5 illustrates results obtained by secondarily differentiating parts of the absorption spectra shown in Fig. 1;

Fig. 6 illustrates absorption spectra of aqueous acetoacetic acid solutions;

Fig. 7 illustrates absorption spectra of aqueous acetone solutions;

Fig. 8 is a block diagram schematically showing an embodiment of a ketone body concentration measuring apparatus; and

Fig. 9 is a block diagram schematically showing another embodiment of a ketone body concentration measuring apparatus.

## DESCRIPTION OF THE PREFERRED EMBODIMENTS

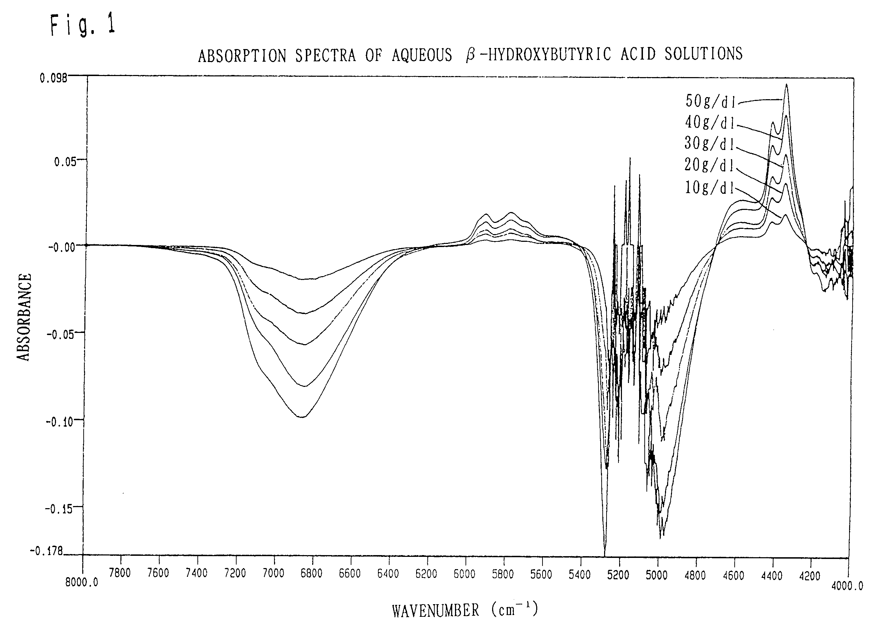

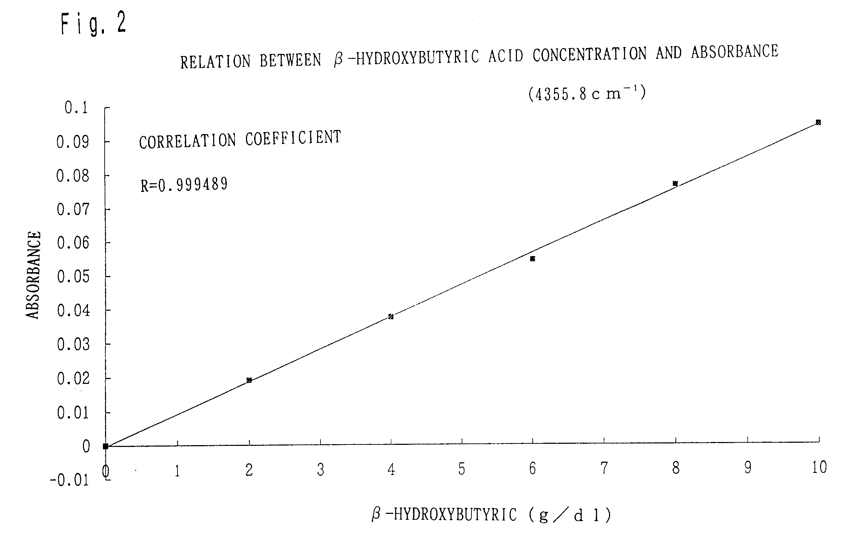

Fig. 1 illustrates absorption spectra of aqueous solutions of β-hydroxybutyric acid, which is an intravascular ketone body, in a near infrared region. The samples are five types of aqueous solutions having concentrations of 10 g/dl, 20 g/dl, 30 g/dl, 40 g/dl and 50 g/dl respectively. Bands changing absorbance values in proportion to the concentrations are recognized everywhere, and these bands are expressed in wavenumbers as 7600 to 5600 $cm^{-1}$ and 4800 to 4200 $cm^{-1}$. A calibration curve obtained by plotting the absorbance values at the characteristic absorption wavenumber of 4355.8 $cm^{-1}$ with respect to the concentrations is that shown in Fig. 2. The linearity of the calibration curve shown in Fig. 2 is 0.999489 when expressed in a correlation coefficient R, and this indicates that the same is excellent as a calibration curve. Such a calibration curve can be formed in a wavenumber region having a correlation coefficient of at least 0.8, preferably at least 0.9.

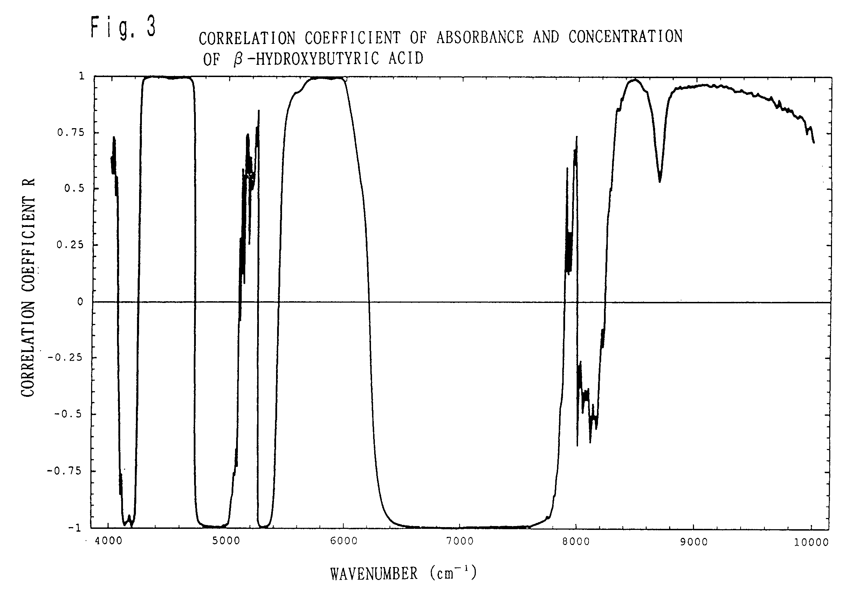

Fig. 3 illustrates results obtained by calculating correlation coefficients R of the absorbance values and the concentrations from the absorption spectra of Fig. 1 as to the respective wavenumbers. High correlation coefficients are obtained in a number of wavenumber bands, and a high accuracy calibration expression of β-hydroxybutyric acid is obtained when a multivariate analytical operation such as PCR or PLS is performed in the wavenumber regions having high correlation coefficients.

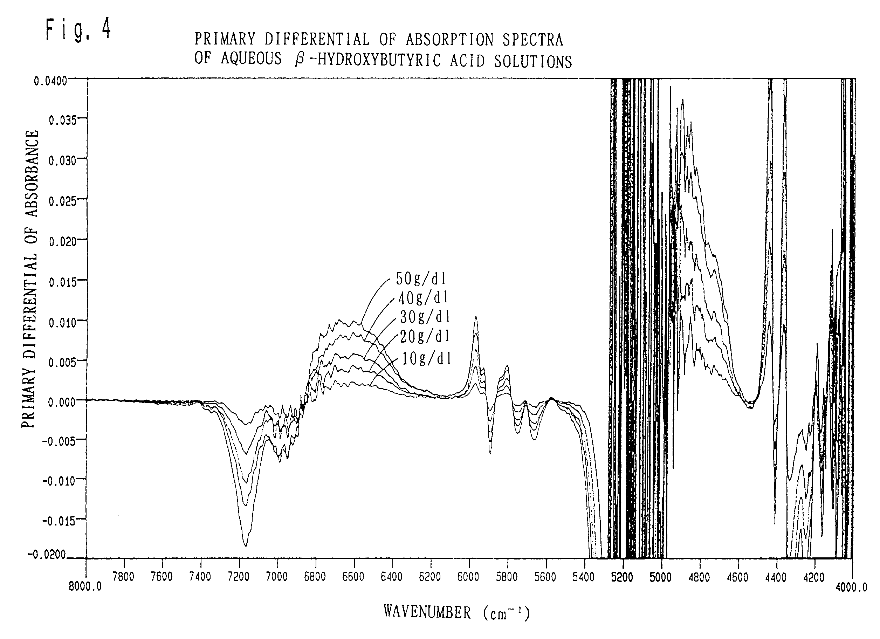

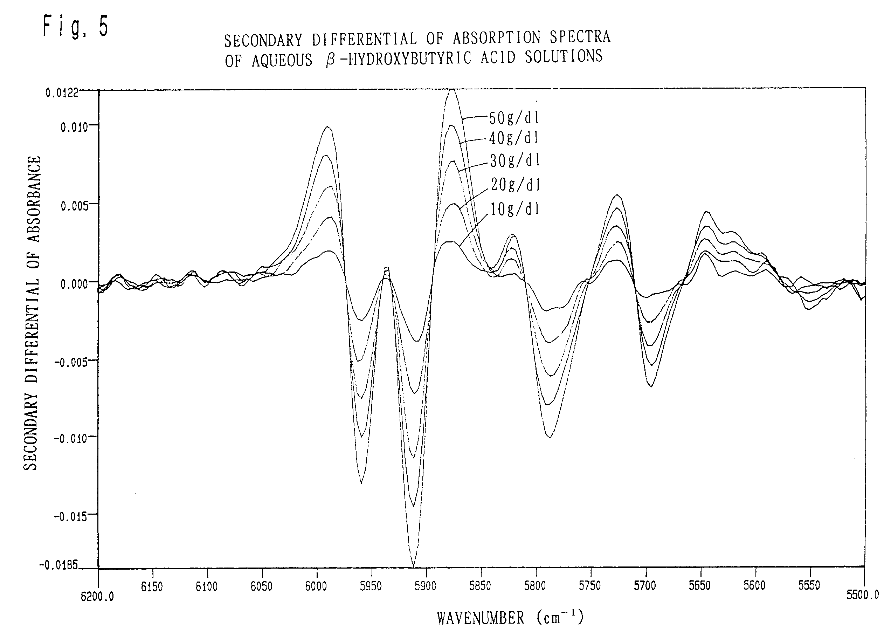

In order to effectively extract a signal which is hard to detect due to influence by an interfering substance, it is effective to perform a multivariate analytical operation after differentiating absorption spectra with respect to wavenumbers or wavelengths. Fig. 4 illustrates results obtained by primarily differentiating parts of the absorption spectra shown in Fig. 1, and Fig. 5 illustrates results obtained by secondarily differentiating parts of the absorption spectra respectively. Also from the results of the primary and secondary differentials, it is clearly understood that spectral intensities are changed in proportion to the concentrations.

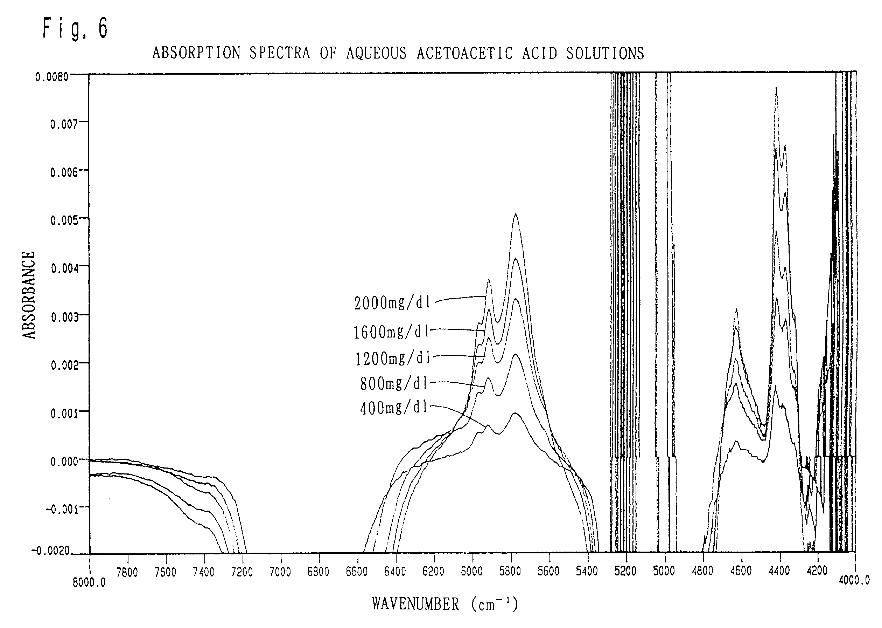

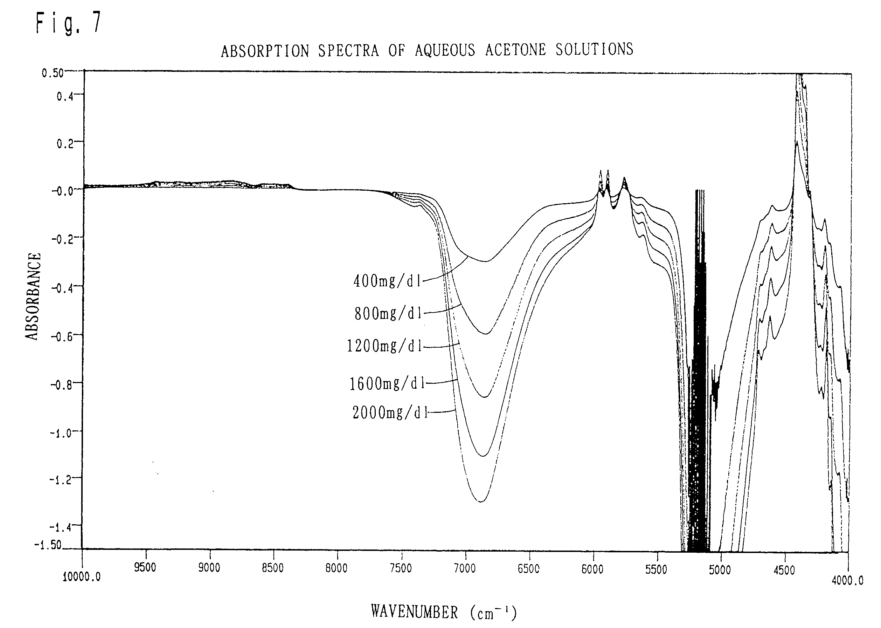

Figs. 6 and 7 illustrate absorption spectra of respective aqueous solutions of acetoacetic acid and acetone which are other intravascular ketone bodies respectively, as to samples having concentrations of 400 mg/dl, 800 mg/dl, 1200 mg/dl, 1600 mg/dl and 2000 mg/dl respectively. It is clearly understood that concentration dependency is exhibited as to both of acetoacetic acid and acetone, and calibration curves with respect to the concentrations and the absorbance values can be formed in wavenumber regions having correlation coefficients of at least 0.8 or 0.9 between the absorbance values and the concentrations. Further, the calibration curves can also be formed by performing primary or secondary differentials, similarly to the case of β-hydroxybutyric acid.

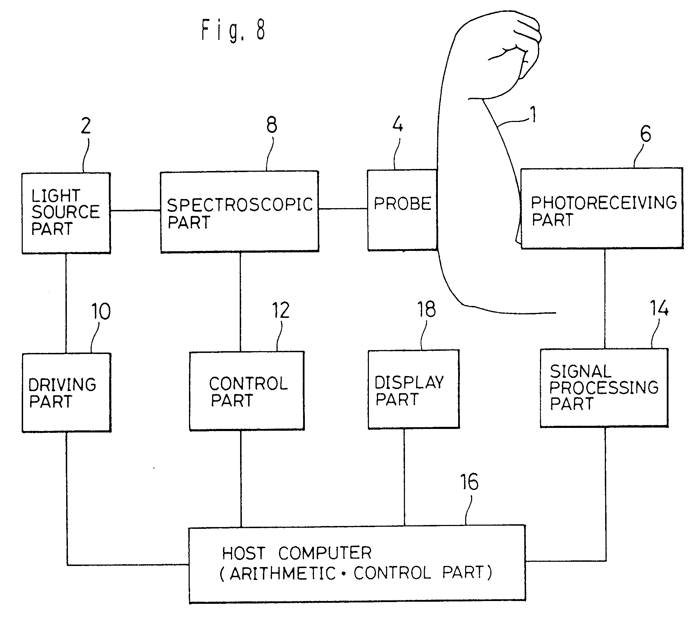

Fig. 8 schematically illustrates an embodiment of an intravital ketone body concentration measuring apparatus according to the present invention. A light source part 2 is formed by a halogen lamp or the like which emits light of a plurality of wavelengths of a near infrared region, and a spectroscopic part 8 is provided on an optical path for irradiating an organism measuring portion 1 with measuring light emitted from the light source part 2 for selecting a wavelength which has excellent correlation of at least 0.8 or at least 0.9 between the concentration of a ketone body to be measured and absorbance and is selected as a measuring wavelength from the measuring light of a plurality of wavelengths emitted from the light source part 2. A probe 4 comes into contact with the organism measuring portion 1, for irradiating the organism measuring portion 1 with the measuring light of the wavelength selected at the spectroscopic part 8. A photoreceiving part 6 detects transmitted/scattered light of the measuring light which is incident upon the organism measuring portion 1.

The spectroscopic part 8 is of the so-called prespectroscopic system of performing wavelength selection before the organism measuring portion 1 is irradiated with the measuring light, while the spectroscopic part 8 may alternatively be provided on an optical path for introducing the transmitted light of the measuring light into the photoreceiving part 6 to be of the so-called postspectroscopic system.

Numeral 10 denotes a driving part for driving the light source part 2, numeral 12 denotes a control part for controlling the wavelength selection of the spectroscopic part 8, and numeral 14 denotes a signal processing part for converting a light signal detected by the photoreceiving part 6 to absorbance. Operations of the respective parts are controlled by a host computer 16 serving as an arithmetic • control part, and the concentration of a ketone body is calculated on the basis of a detected transmitted/scattered light intensity. The result of the obtained ketone body concentration is displayed on a display part 18.

The spectroscopic part 8 may be a monochromatic spectroscope comprising a prism or a diffraction grating, or a Fourier transform spectrophotometer comprising a Michelson interferometer and a Fourier transform arithmetic part in the arithmetic • control part 16. Alternatively, the spectroscopic part 8 may comprise an AOTF (acousto-optical filter), and a high-speed operation is enabled in this case. Further, the spectroscopic part 8 may comprise a plurality of optical filters, for selecting the measuring wavelength by being switched and positioned on the optical path.

The probe 4 is adapted to guide the measuring light to the organism measuring portion 1 by optical fiber, for example. The measuring light is applied from the probe 4, and the light which is transmitted/scattered through/from the organism measuring portion 1 and incident upon the photoreceiving part 6 is strongly scattered by the organism. In order to receive such scattered light, therefore, the photoreceiving part 6 preferably comprises an integrating sphere so that the scattered light collected by the integrating sphere is detected.

The spectroscopic part 8 is necessary when the light source part 2 is formed by a light source such as a lamp emitting light of continuous wavelengths or multiple wavelengths, while the spectroscopic part 8 is unnecessary when the light source part 2 is formed by a laser unit, a laser diode or a light emitting diode emitting single wavelength light. For example, the light source part 2 may comprise a laser diode or a light emitting diode, or a plurality of types of laser diodes or light emitting diodes having different light emission wavelengths. It is possible to irradiate the organism measuring portion 1 with measuring light of specific wavelengths in a successively switched manner by switching and turning on light emission of these laser diodes or the like. Thus, absorbance measurement with multiple wavelengths can be performed without employing the spectroscopic part 8.

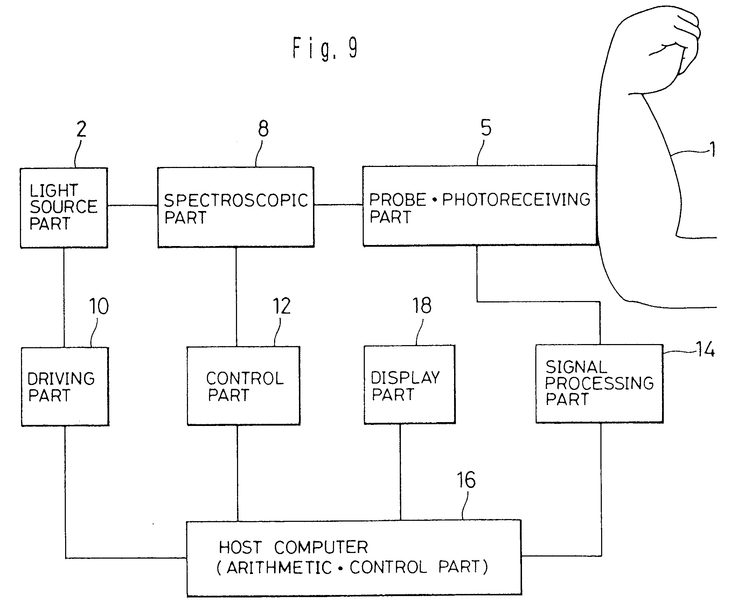

Fig. 9 shows another embodiment. While the transmitted/scattered light of the measuring light from the probe 4 is received by the photoreceiving part 6 in the embodiment shown in Fig. 8, a probe and a photoreceiving part are integrated with each other to form a probe • photoreceiving part 5 in the embodiment shown in Fig. 9. Transmitted/scattered light of measuring light from the probe • photoreceiving part 5 is detected by the probe • photoreceiving part 5. Other structure of this embodiment is identical to that shown in Fig. 8.

Although the present invention has been described and illustrated in detail, it is clearly understood that the same is by way of illustration and example only and is not to be taken by way of limitation, the spirit and scope of the present invention being limited only by the terms of the appended claims.

## Claims

1. A measuring method of irradiating an organism with near infrared light for quantitatively analyzing a ketone body concentration in an organism through light being transmitted/scattered through/from said organism.

2. The measuring method in accordance with claim 1, wherein
   a light intensity at a measuring wavelength being subjected to absorption by a ketone body in a near infrared region is detected from said light being transmitted/scattered through/from said organism, for quantitatively analyzing said intravital ketone body concentration on the basis of said light intensity.

3. The measuring method in accordance with claim 2, wherein
   said measuring wavelength is a wavelength having a correlation coefficient of at least 0.8, preferably at least 0.9, between said ketone body concentration and absorbance in aqueous ketone body solution measurement.

4. The measuring method in accordance with claim 3, wherein
   said ketone body to be measured includes at least one of acetoacetic acid, $\beta$-hydroxybutyric acid and acetone, and said measuring wavelength for each said component, belonging to a near infrared region being expressed in wavenumbers of 10000 to 4000 $cm^{-1}$, is:

   selected from around 6400 to 5400 or 4800 to 4300 $cm^{-1}$ for acetoacetic acid,
   selected from around 7600 to 5600 or 4800 to 4200 $cm^{-1}$ for $\beta$-hydroxybutyric acid, or
   selected from around 7800 to 5500 or 4800 to 4200 $cm^{-1}$ for acetone.

5. The measuring method in accordance with claim 1, wherein
   light intensities at a plurality of measuring wavelengths being subjected to absorption by said ketone body in a near infrared region are detected from said light being transmitted/scattered through/from said organism for quantitatively analyzing said intravital ketone body concentration from said light intensities through multivariate analysis.

6. The measuring method in accordance with claim 5, wherein

said measuring wavelengths have correlation coefficients of at least 0.8, preferably at least 0.9 between ketone body concentrations and absorbance values in aqueous ketone body solution measurement.

7. The measuring method in accordance with claim 6, wherein

said ketone body to be measured includes at least one component among acetoacetic acid, β-hydroxybutyric acid and acetone, and a measuring wavelength for each said component, belonging to a near infrared region being expressed in wavenumbers of 10000 to 4000 cm$^{-1}$, is:

selected from around 6400 to 5400 or 4800 to 4300 cm$^{-1}$ for acetoacetic acid,
selected from around 7600 to 5600 or 4800 to 4200 cm$^{-1}$ for β-hydroxybutyric acid, or
selected from around 7800 to 5500 or 4800 to 4200 cm$^{-1}$ for acetone.

8. A measuring apparatus for ketone body in organism comprising:

a light source part (2) emitting wavelength light in a near infrared region;
a probe (4, 5) for coming into contact with an organism measuring portion (1) for irradiating said organism measuring portion (1) with said light from said light source part (2) as measuring light;
a photoreceiving part (6, 5) for detecting transmitted/scattered light of said measuring light being received from said organism measuring portion (1); and
an arithmetic part (16) for calculating a ketone body concentration on the basis of one or a plurality of measuring wavelengths, detected by said photoreceiving part (6, 5), being subjected to absorption by ketone body.

9. The measuring apparatus in accordance with claim 8, wherein

said light source part (2) emits light of a plurality of wavelengths of said near infrared region,
said apparatus further comprising a spectroscopic part (8) being provided on an optical path for irradiating said organism measuring portion (1) with said measuring light or an optical path for introducing said transmitted/scattered light of said measuring light into said photoreceiving part (6, 5) for selecting one or a plurality of wavelengths being subjected to absorption by ketone body as said measuring wavelength(s) from said light of said plurality of wavelengths being emitted from said light source part (2).

10. The measuring apparatus in accordance with claim 8, wherein

said light source part (2) comprises any of a laser unit, a laser diode and a light emitting diode emitting light of said wavelength(s) of said near infrared region being said wavelength(s) subjected to absorption by ketone body and selected as said measuring wavelength(s).

11. The measuring apparatus in accordance with claim 10, wherein

said light source part (2) includes a plurality of types of laser diodes or light emitting diodes having different emission wavelengths for successively generating said measuring light of a plurality of wavelengths by being successively switched and turned on.

Fig. 1

ABSORPTION SPECTRA OF AQUEOUS $\beta$-HYDROXYBUTYRIC ACID SOLUTIONS

EP 0 762 108 A2

# Fig. 2

RELATION BETWEEN $\beta$-HYDROXYBUTYRIC ACID CONCENTRATION AND ABSORBANCE

$(4355.8\,c\,m^{-1})$

CORRELATION COEFFICIENT

R=0.999489

ABSORBANCE (vertical axis)

$\beta$-HYDROXYBUTYRIC (g／d l)

Fig. 3     CORRELATION COEFFICIENT OF ABSORBANCE AND CONCENTRATION
           OF $\beta$-HYDROXYBUTYRIC ACID

EP 0 762 108 A2

Fig. 4

PRIMARY DIFFERENTIAL OF ABSORPTION SPECTRA
OF AQUEOUS $\beta$-HYDROXYBUTYRIC ACID SOLUTIONS

50g/dl
40g/dl
30g/dl
20g/dl
10g/dl

PRIMARY DIFFERENTIAL OF ABSORBANCE

WAVENUMBER (cm$^{-1}$)

Fig. 5

SECONDARY DIFFERENTIAL OF ABSORPTION SPECTRA
OF AQUEOUS β−HYDROXYBUTYRIC ACID SOLUTIONS

EP 0 762 108 A2

Fig. 6

ABSORPTION SPECTRA OF AQUEOUS ACETOACETIC ACID SOLUTIONS

2000mg/dl
1600mg/dl
1200mg/dl
800mg/dl
400mg/dl

ABSORBANCE

WAVENUMBER $(cm^{-1})$

Fig. 7

ABSORPTION SPECTRA OF AQUEOUS ACETONE SOLUTIONS

WAVENUMBER (cm$^{-1}$)

EP 0 762 108 A2

Fig. 8

EP 0 762 108 A2

Fig. 9

EP 0 762 108 A2